# EUROPEAN PATENT APPLICATION

(11) **EP 0 576 953 A2**
(43) Date of publication of application: **05.01.1994**
(21) Application number: 93109879.2
(22) Date of filing: 21.06.1993
(51) Int. Cl.: C12N 15/57, C12N 9/64, C12N 5/10, A61K 37/54

(54) **Production of human cathepsin and its use**

(30) Priority: 19.06.1992 JP 185947/92; 02.06.1993 JP 157827/93
(71) Applicant: TAKEDA CHEMICAL INDUSTRIES, LTD., Chuo-ku, Osaka 541 (JP)
(72) Inventor: Fujisawa, Yukio, Kobe, Hyogo 658 (JP); Katsunuma, Nobuhiko, Tokushima 770 (JP); Tajima, Naoya, Miyazaki 889-21 (JP)
(74) Representative: Casalonga, Axel

(57) **Abstract**

The present invention relates to a mouse myeloma cell carrying a DNA encoding human cathepsin, a method of producing human cathepsin by culturing said cell and a therapeutic composition for intervertebral disk hernia.

## Description

### Field of the invention

The present invention relates to a method of producing human cathepsin and a therapeutic composition for intervertebral disk hernia containing said enzyme.

### Background of the invention

Intervertebral disk hernia, a disease likely to develop in the lower lumbar vertebrae and the cervical vertebrae, is accompanied by sciatic neuralgia, upper limb neuralgia and other symptoms. With high compression on intervertebral disks after initial degeneration and loss of fibrous ring elasticity, the vertebral pulp is pushed out from fibrous ring cracks. This condition, called intervertebral disk hernia, results in pain and other symptoms caused by inflammation in and around the nerve root, when the herniated intervertebral disks have compressed the equine caudal spine and the nerve root branched out from the spine.

Traditionally, intervertebral disk hernia has been treated by various methods of pain relief: by administration of anti-inflammatory sedatives, muscular relaxants and other drugs in the acute phase attended by severe pain; and, in the chronic phase, by traction, thermotherapy and kinesitherapy. However, surgical treatment is often adopted in cases where the more conservative therapy fails.

In the acute phase of intervertebral disk hernia, a conservative therapy is supplemented with an oral anti-inflammatory sedative and rest, as there is severe pain. Even after mitigation of the severe pain and inflammation in the acute phase, the hernia does not disappear. Other conservative therapies include traction, thermotherapy in which painful regions in the lumbar and feet are warmed to relax stiff muscles, and lumbar pain exercise for strengthening abdominal and dorsal muscles. In cases where the conservative therapy fails, intervertebral disks are excised by surgery, as stated above.

In some country, particularly in the United States and Canada, chymopapain, a protease of plant origin, is used to treat intervertebral disk hernia. This aims at mitigating compression on the nerve tissue by chemically lysing intervertebral disks and reducing the internal pressure thereof. However, the nature of chymopapain as a foreign protein poses the problem of side effects such as anaphylactic shock. To solve this problem, there is a need for the development of a more effective drug with a lower prevalence of toxicity.

At present, the only available method of producing human cathepsin is expression of a cDNA encoding human cathepsin L in *Escherichia coli*, there being no production method employing any other host such as an animal cell.

Through intensive investigation, the present inventors found that human cathepsin has a therapeutic effect on intervertebral disk hernia. The inventors demonstrated that human cathepsin L or S produced in animal cells by gene engineering and cathepsin L or S derived from human cell lines are suitable for treatment of intervertebral disk hernia, and developed the present invention.

### Summary of the Invention

The present invention provides a therapeutic composition for intervertebral disk hernia containing human cathepsin. The invention also provides a method of producing human cathepsin, wherein animal cells carrying a DNA encoding human cathepsin are cultured to produce and accumulate said enzyme in the culture, which is then harvested.

### Brief Description of Drawings

Figure 1 shows a construction scheme for the plasmids obtained in Examples 1 and 4.

Figure 2 shows production of human cathepsin L, by a COS-7 cell transformant, observed in Example 2.

Figure 3 shows production of human cathepsin L, by an Sp2/0 cell transformant, observed in Example 5. In the figure, panels A and B respectively show the results of western blotting and CBB staining.

Figure 4 is a toluidine blue stained image obtained after a dose of 1600milliunits cathepsin L in Example 7.

Figure 5 is a toluidine blue stained image obtained after a dose of 800milliunits cathepsin L in Example 7.

Figure 6 is a toluidine blue stained image obtained after a dose of 400milliunits cathepsin L in Example 7.

Figure 7 is a toluidine blue stained image obtained after a dose of 200milliunits cathepsin L in Example 7

Figure 8 is a hematoxylin-eosin stained image obtained after a dose of 1600milliunits cathepsin L in Example 7.

Figure 9 is a hematoxylin-eosin stained image obtained after a dose of 800milliunits cathepsin L in Example 7.

Figure 10 is a hematoxylin-eosin stained image obtained after a dose of 400milliunits cathepsin L in Example 7.

Figure 11 is a hematoxylin-eosin stained image obtained after a dose of 200milliunits cathepsin L in Example 7.

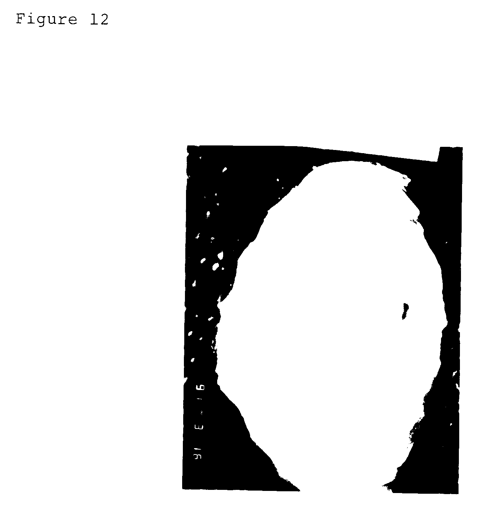

Figure 12 is a macroscopic view of a normal rabbit intervertebral disk.

Figure 13 is a macroscopic view of a rabbit intervertebral disk after a dose of 800milliunits cathepsin L in Example 7.

Figure 14 shows a construction scheme for the plasmid obtained in Example 9.

### Detailed Description

Human cathepsins used in the present invention include cathepsins A, B, C, D, E, F, G, H, L, S and T. These human cathepsins may be of a natural type or a mutein. Among them, the use of those having a molecular weight of about 20,000 to about 40,000 is preferable. In the preparation of the present invention, preference was given to cathepsin L or S. The human cathepsin L or S may be a product of either gene engineering technology or a human cell line.

Muteins of human cathepsin include those resulting from mutation in the amino acid sequence of the original protein except all the eight cystein residues and the 163rd histidine residue produced by amino acid addition, constituent amino acid deletion, or replacement with another amino acid. Amino acid addition may be such that a mutein results from the addition of at least one amino acid to human cathepsin, excluding methionine or signal peptides from the initiation codon used to express the peptide, as long as the human cathepsin activity is maintained. Deletion of constituent amino acids may be such that a mutein results from a lack of at least one constituent amino acid of human cathepsin, as long as the activity of human cathepsin is retained. Replacement with another amino acid may be such that a mutein results from replacement of at least one human cathepsin constituent amino acid with another amino acid, as long as the activity of human cathepsin is retained.

Said mutein may involve a combination of two or more of addition, deletion and replacement. Such deletion or replacement is generally carried out in position(s) of 1 to 3 amino acid residue(s) of the original protein. Such mutein is preferably a mutein of human cathepsin L or S.

As mentioned above, the human cathepsin and mutein thereof in the present invention can be derived from a human cell line producing human cathepsin selected by established human cell line screening. They can also be produced by gene engineering technology using a cDNA encoding human cathepsin. For example, it is possible to use a reported cDNA encoding human cathepsin [S. Gal and M. M. Gottesman, Biochem. J., 253, 303 (1988)]. In the production method of the present invention, human cathepsin can be obtained by expressing said cDNA in cultured animal cells. Accordingly, the present invention comprises (1) synthesis of a PCR primer to amplify a reported nucleotide sequence of cathepsin of human renal origin, (2) preparation of a recombinant DNA to express the amplified human cathepsin gene using cultured animal cells, (3) establishment of an animal cell line transformed with the recombinant DNA described in (2) above, and (4) separation and purification of human cathepsin from a culture of the animal cell line described in (3) above.

In the presence of a sense primer and antisense primer synthesized on the basis of the above-mentioned reported nucleotide sequence, a polymerase chain reaction (PCR) can be conducted in accordance with the known method, such as the instructions for a kit (e.g., Cetus/Perkin-Elmer). The amplified cDNA can be separated by a commonly known method such as agarose electrophoresis and then recovered from the gel. The nucleotide sequence of this cDNA can be determined by the dideoxynucleotide synthetic chain termination method [T. Messing et al., Nucl. Acids Res., 9, 309 (1981)]. The plasmid harboring the cloned cDNA can be used as it is or after being cut out with an appropriate restriction enzyme and then inserted into another vector as necessary.

Any vector can be used, as long as it is replicable in the host. When the host is a bacterium of the genus *Escherichia* (e.g., *Escherichia coli*), such vectors include plasmids derived from *Escherichia coli* such as pBR322 [F. Bolivar et al., Gene, 2, 95 (1979)], pBR325, pUC12 and pUC13. When the host is a bacterium of the genus *Bacillus*, such vectors include plasmids derived from *Staphylococcus* such as pUB110 [T. J. Gryczan and D. Dubnau, Proc. Natl. Acad. Sci. USA, 75, 128 (1978)], pTP5 [N. Noguchi, Gene, 2, 95 (1979)] and pC194 [D. Dubnau, Experimental Manipulation of Gene Expression; ed. M. Inouye, p. 83, Academic Press, (1983)]. When the host is a yeast, such vectors include plasmids derived from yeast such as pSH19 [S. Harashima et al., Mol. Cell. Biol., 4, 771 (1984)] and pSH19-1 [European Patent Publication EP-A-0235430]. When the host is an animal cell, such vectors include pSV2-X, resulting from insertion of SV40 ori into pBR322 [R. C. Mulligan and P. Berg, Proc. Natl. Acad. Sci. USA, 78, 2072 (1981)] and pcD-X [H. Okayama and P. Berg, Mol. Cell. Biol., 3, 280 (1983)].

The cloned cDNA may have a translational initiation codon (ATG) at its 5'-terminal and a translational termination codon (TAG, TGA or TAA) at its 3'-terminal. To express the cDNA, a promoter sequence, promoter-pre-pro sequence or promoter-signal sequence is ligated upstream.

Any promoter can be used for the present invention, as long as it is appropriate for the host used to express the gene. Examples of promoters are: when the host is *Escherichia coli*, the trp promoter, lac promoter, λ PL promoter, T7 promoter, tac promoter, lpp promoter and recA promoter, with preference given to the T7 promoter; when the host is a bacterium of the genus *Bacillus*, the SPO1 promoter, SPO2 promoter, penP promoter and MWP promoter, with preference given to the MWP promoter; when the host is a yeast, the GAPDH promoter, PGK promoter, PHO5 promoter, ADH promoter and PHO81 promoter, with preference given to the GAPDH promoter; and when the host is an animal cell, the SV40-derived promoter, retrovirus promoter and human cytomegalovirus promoter. The promoter can be prepared from the corresponding gene or chemically synthesized by known methods.

The signal sequence and pre-pro sequence may be any one, as long as they function in the host. In the case of *Escherichia coli*, they include the β-lactamase gene signal sequence, enterotoxin gene signal sequence, alkaline phosphatase gene signal sequence and OmpA gene signal sequence. In the case of a bacterium of the genus *Bacillus*, they include the α-amylase gene signal sequence, neutral protease gene signal sequence and MWP gene signal sequence. In the case of a yeast, they include the egg white lysozyme gene signal sequence, human lysozyme gene signal sequence, invertase gene signal sequence and α-factor gene pre-pro sequence. In the case of an animal cell, they include the interleukin-2 gene signal sequence.

The DNA-harboring vector thus constructed is used to produce a transformant. Examples of the host include bacteria of the genus *Escherichia*, bacteria of the genus *Bacillus*, yeasts and animal cells, with preference given to animal cells. Examples of the bacteria of the genus *Escherichia* include *Escherichia coli* K12 DH1 [B. Low, Proc. Natil. Acad. Sci. USA 60, 160 (1968)], C600 [R. K. Appleyard, Genetics, 39, 440 (1954)], MM294 [K. Backman et al., Proceedings of the Natl. Acad. Sci. USA, 73, 4174 (1976)] and N4830 [M. E. Gottesman et al., J. Mol. Biol., 140, 57 (1980)]. Examples of the bacteria of the genus *Bacillus* include *Bacillus subtilis* MI114 [K. Yoshimura et al., Gene 24, 255 (1983)], 207-21 [K. Ohmura et al., J. Biochem., 95, 87 (1984)] and *Bacillus brevis* 47 [S. Udaka, Agric. Biol. Chem., 40, 523 (1976)]. Examples of the yeasts include *Saccharomyces cerevisiae* AH22R⁻ [A. Miyanohara et al., Proc. Natl. Acad. Sci. USA, 80, 1 (1983)], NA87-11A, DKD-5D, NA74-3A, NA74-3Ap⁻ [Y. Kaisho et al., Yeast 5, 91 (1989)], *Schizosaccharomyces pombe* ATCC38399 (h-leul-32) and TH168 (h90 ade6-M210 ura1 leu1) [M. Kishida and C. Shimada, Current Genetics, 10, 443 (1986)]. Examples of animal cells include simian COS-7 cells, simian Vero cells, Chinese hamster (CHO) cells, mouse L cells, human FL cells, all of which are adherent cells, and mouse myeloma cells (e.g., Sp2/0), mouse YAC-1 cells, mouse MethA cells, mouse P388 cells and mouse EL-4 cells, all of which are suspending cells.

Bacteria of the genus *Escherichia* can be transformed in accordance with the method described by T. Maniatis et al. in Molecular Cloning, Cold Spring Harbor Laboratory, page 249 (1982) and other publications, for instance. Bacteria of the genus *Bacillus* can be transformed in accordance with the method described by S. Chang and S. N. Cohen in Molecular and General Genetics, 168, 111 (1979) and other publications, for instance. Yeasts can be transformed in accordance with the method described by A. Hinnen et al. in the Proceedings of the National Academy of Science, USA, 75, 1929 (1978), for instance. Animal cells can be transformed in accordance with the method described by M. Wigler et al. in Cell, 14, 725 (1978), for instance.

The transformant thus obtained is cultured by a commonly known method. Examples of media preferably used to cultivate a transformant whose host is an *Escherichia* bacteria include the M9 medium containing glucose and casamino acid [J. H. Miller, Experiments in Molecular Genetics, page 431, Cold Spring Harbor Laboratory, (1972)]. To increase promoter efficiency as necessary, a chemical agent such as isopropylthiogalactoside (IPTG) or indolyl 3-acrylate may be added. Cultivation is normally carried out at about 15 to 43°C for about 3 to 24 hours, with aeration and/or stirring as necessary.

Examples of media used to cultivate a transformant whose host is a *Bacillus* bacteria include the L-broth medium and T2 medium [S. Udaka, Agric. Biol. Chem., 40, 523 (1976)]. Cultivation is normally carried out at about 15 to 37°C for about 6 to 96 hours, with aeration and/or stirring as necessary.

Examples of media for cultivating a transformant whose host is a yeast include Burkholder's minimal medium [K. L. Bostian et al., Proc. Natl. Acad. Sci. USA, 77, 4504 (1980)]. It is preferable to adjust the medium to a pH level of about 5 to 8. Cultivation is normally carried out at about 20 to 35°C for about 24 to 72 hours, with aeration and/or stirring as necessary.

Examples of media for cultivating a transformant whose host is an animal cell include MEM medium containing about 5 to 20% fetal bovine serum [H. Eagle, Science, 130, 432 (1959)], DMEM medium [R. Dulbecco and G. Freeman, Virology, 8, 396 (1959)], RPMI-1640 medium [G. E. More et al., J. Am. Med. Assoc., 199, 519 (1967)], 199 medium [J. F. Morgan et al., Proc. Soc. Exp. Biol. Med. 73, 1 (1950)] and ASF104 medium [Y. Minamoto, Cell Technology, 7, 42 (1988)]. Cultivation is normally carried out at about 30 to 40°C for about 15 to 60 hours, with aeration and/or stirring as necessary.

To isolate the expression product from the above culture, commonly known methods of separation and purification can be used in combination as appropriate. Such commonly known methods of separation and purification include those based on solubility differences such as salting-out and solvent precipitation, those based mainly on molecular weight differences such as dialysis, ultrafiltration, gel filtration and SDS-polyacrylamide gel electro-phoresis (SDS-PAGE), those based on charge differences such as ion exchange chromatography, those based on specific affinity such as affinity chromatography, those based on hydrophobicity differences such as reverse-phase high performance liquid chromatography, and those based on isoelectric point differences such as isoelectric focusing.

As described in the following examples, the present invention has made it possible to express human cathepsin by gene engineering using animal cells, enabling production of human cathepsin of high purity in large amounts.

As therapeutic agents for intervertebral disk hernia, human cathepsin can be administered alone or in a pharmaceutical composition with pharmacologically acceptable carriers, excipients and diluents to mammals (e.g., dogs, cats, bovines, horses, humans). The most suitable route of administered for intervertebral disk hernia is direct injection to the intervertebral pulp via a posterior lateral approach. This promotes the safety of treatment. The pharmaceutical composition of the present invention, which uses the cathepsin derived from human, is particularly advantageous because of its low antigencity. Preferred dosage forms include injections, solutions for injection, frozen products and freeze-dried products.

In preparing a pharmaceutical composition, pharmaceutically acceptable additives, diluents, excipients, etc. are used as necessary, in accordance with commonly accepted pharmaceutical production methods.

For example, an aqueous solution for injection is prepared by a conventional method using a solvent such as an aqueous solvent (e.g., distilled water), a water-soluble solvent (e.g., physiological saline, Ringer's solution) or an oily solvent (e.g., sesame oil, olive oil), and if desired, additives such as dissolution aids (e.g., sodium salicylate, sodium acetate), buffers (e.g., sodium citrate, glycerol), isotonizing agents (e.g., glucose, invert sugar), stabilizers (e.g., human serum albumin, polyethylene glycol), preservatives (e.g., benzyl alcohol, phenol) and soothing agents (e.g. benzalkonium chloride, procaine hydrochloride).

As another example, a composition for injection is prepared in a solid form of tablet, powder or granule powder by conventional methods such as a mixing process of powdered cathepsin and pharmaceutically acceptable adjuvant or freeze-drying of solution containing cathepsin and pharmaceutically acceptable adjuvant. The pharmaceutically acceptable adjuvant includes excipients (e.g., carboxymethyl cellulose (CMC), sodium alginate), preservatives (e.g., benzyl alcohol, benzalkonium chloride, phenol) and soothing agents (e.g., glucose, calcium gluconate, procaine hydrochloride).

When used this solid composition is dissolved or suspended in an aqueous solution e.g. distilled water, physiological saline, glucose solution.

In making a composition, monosaccharides such as glucose, amino acids, various salts, human serum albumin etc. may be added, and isotonizing agents, pH regulators, soothing agents, antiseptics and other additives may be added for a safe and effective preparation of human cathepsin L or a mutein thereof.

More preferably, cathepsin L is dissolved in 0.5 M acetate bufffer (pH 4.0) or supplemented with human serm albumin (HSA/cathepsin L (mol) = 100) to stabilize the enzyme.

Although the preparation dose of the present invention for injection into intervertebral pulp can be determined as appropriate to the intervertebral disk hernia symptoms, it is preferable to administer it at 100 to 500,000milliunits, and more preferably about 1,000 to 100,000milliunits, per administration. Herein, one unit corresponds to 1 mg of substrate degarded per minute at 37°C (Method in Enzmology, 80, 535 (1981)). Administration frequency may be once a day or at longer intervals, once a week, for instance.

Thus cultivation of animal cells harboring a DNA encoding human cathepsin according to the method of the present invention makes it possible to produce said enzyme at high purities and in large amounts. Also, the composition containing human cathepsin of the present invention appropriately lyses mammalian intervertebral pulp and can therefore be used to treat intervertebral disk hernia. The composition of the present invention is useful as a therapeutic agent for intervertebral disk hernia.

Abbreviations for bases, amino acids and other abbreviations used in the present specification and in drawings attached thereto are specified by the IUPAC-IUB Commission on Biochemical Nomenclature or they are in common use in relevant fields. Some examples are given below. When an optical isomer may be present in an amino acid, it is of the L-configuration, unless otherwise stated.
- DNA: : Deoxyribonucleic acid
- A: : Adenine
- T: : Thymine
- G: : Guanine
- C: : Cytosine
- SDS: : Sodium dodecyl sulfate
- Gly: : Glycine (G)
- Ala: : Alanine (A)
- Val: : Valine (V)
- Leu: : Leucine (L)
- Ile: : Isoleucine (I)
- Ser: : Serine (S)
- Thr: : Threonine (T)
- Cys: : Cysteine (C)
- 1/2 Cys: : Half cysteine
- Met: : Methionine (M)
- Glu: : Glutamic acid (E)
- Asp: : Aspartic acid (D)
- Lys: : Lysine (K)
- Arg: : Arginine (R)
- His: : Histidine (H)
- Phe: : Phenylalanine (F)
- Tyr: : Tyrosine (Y)
- Trp: : Tryptophan (W)
- Pro: : Proline (P)
- Asn: : Asparagine (N)
- Gln: : Glutamine (Q)
- Apr: : Ampicillin resistance gene
- Tcr: : Tetracycline resistance gene

### Examples

The present invention is hereinafter described in more detail by means of the following reference examples and working examples, which are not to be construed as an exhaustive explication of the full scope of the present invention.

The microorganism and animal cell obtained in the following Examples have been deposited under accession numbers shown in the following table.

| | IFO IFO No. | FRI FERM BP-No. |
|---|---|---|
| E. coli JM 109/pTBN-HCL neo | 15341 (1992.6.12) | 3897 (1992.6.22) |
| Mouse myeloma Sp-HCL26 | 50371 (1992.6.16) | 3902 (1992.6.24) |
| Mouse myeloma Sp-HCS30 | 50401 (1993.4.21) | 4289 (1993.4.28) |
| Mouse myeloma Sp-HCL26-1 | 50402 (1993.4.21) | 4288 (1993.4.28) |
| IFO: Institute for Fermentation, Osaka: 17-85, Jusohonmachi 2-chome, Yodogawa-ku, Osaka 532 Japan FRI: Fermentation Research Institute Agency of Industrial Science and Technology: 1-3, Higashi 1-chome, Tsukuba-shi, Ibaraki-ken 305 Japan | | |

In the table, the deposit date is shown in ( ).

### Reference Example 1: Cloning of cathepsin L cDNA of human renal origin

To amplify human cathepsin cDNA by the PCR method, four primers were synthesized in accordance with a reported base sequence of cathepsin L of human renal origin [S. Gal and M. M. Gottesman, Biochem. J., 253, 303 (1988)] as follows: 
Sense primer No. 1:
5'-TTTTCAGGGGGCAGTAAGAT-3'
(Sequence ID No. 1)
Sense primer No. 2:
5'-pCCGGATCCGGCTTTTTAGGATTGGTCTA-3'
(Sequence ID No. 2)
Antisense primer No. 3:
5'-GGGGGCTGGTAGACTGAAGA-3'
(Sequence ID No. 3)
Antisense primer No. 4:
5'-pCCGGATCCATTCCTCCCATGCATGCGCC-3'
(Sequence ID No. 4)
3 µl of a solution of the human renal cDNA library λ gt11 (CLONTECH Laboratories, Inc.) and 50 µl of distilled water were mixed. After incubation at 95°C for 5 minutes, the mixture was immediately cooled in ice. Two primers (Nos. 1 and 3 above, 50 pmol of each) were added, and PCR was carried out as per the instruction manual for the kit supplied from Cetus/Perkin-Elmer, in which a series of reactions at 94°C for 1 minute, 55°C for 2 minutes and 72°C for 3 minutes was repeated for 50 cycles. Added to the reaction mixture were two other primers (Nos. 2 and 4 above, 50 pmol of each), and PCR was carried out in the same manner as above. The PCR product was separated by electrophoresis on 1.2% agarose gel; an amplified DNA fragment was observed at a position corresponding to the size (1132 bp) expected from the nucleotide sequence of cathepsin L of human renal origin. This DNA fragment was recovered from the gel and subcloned to the plasmid vector pBluescript^{R} II SK + (produced by STRATAGENE). The nucleotide sequence of the cDNA portion was determined by the dideoxynucleotide synthetic chain termination method [J. Messing et al., Nucleic Acids Res. 9, 309 (1981)] and proved identical with the reported sequence. The plasmid containing this cDNA fragment was named pHCL-5.

### Reference Example 2: Expression of human cathepsin L in Escherichia coli MM294(DE3)

The cDNA obtained in Reference Example 1 was cleaved with restriction enzyme EcoRI and a 798 bp fragment (which encodes a part of the of human pro-cathepsin L and the whole mature region) was recovered. To both ends of this fragment was ligated a BamHI linker (5'-pCCCGGATCCGGG-3'; sequence ID No. 5), and the ligation product was inserted into the plasmid vector pET-3c for expression in *Escherichia coli* [Methods in Enzymology, ed. D. V. Goeddel, 185, 68, Academic Press (1990)]. The plasmid thus constructed was designated pET-HCLα. *Escherichia coli* MM294(DE3) was transformed with pET-HCLα to express human cathepsin L in the presence of the T7 promoter [Methods in Enzymology, 185, 60 (1990)]. The transformant was cultured and cells were disrupted by sonication and subjected to SDS-PAGE; a unique band appeared near 30 kDal, corresponding to human cathepsin L. Since the expressed product formed an inclusion body, human cathepsin L was partially purified from the precipitated fraction of the ultrasonically disrupted transformant.

### Reference Example 3: Preparation of antiserum to recombinant human cathepsin L

The partially purified recombinant human cathepsin L obtained in Reference Example 2 was mixed with an equal amount of Freund's complete adjuvant and a rabbit was inoculated with about 1 ml. Later, a mixture of a partially purified human cathepsin L and an equal amount of Freund's incomplete adjuvant was injected thrice at 10-day intervals, and blood was collected seven days after the final injection. The blood obtained was kept standing at 37°C for 30 minutes and then at 4°C overnight, after which it was centrifuged to yield an antiserum to human cathepsin L.

### Reference Example 4: Cloning of cDNA of cathepsin S of human placental origin

To amplify a human cathepsin S cDNA by the PCR method, four primers were synthesized in accordance with a reported base sequence of human cathepsin S [B. Wiederanders et al., J. Biol. Chem., 267, 13708 (1992)] as follows: 
Sense primer No. 5:
5'-pTAATTCAAGGACTCTTACTG-3'
(Sequence ID No. 6)
 
Sense primer No. 6:
5'-pGGAATTCCTAGGAGCAACTGCTGGTTCT-3'
(Sequence ID No. 7)
Antisense primer No. 7:
5'-pGGAATTCCTCATATTTCATTGATTTGTT-3'
(Sequence ID No. 8)
Antisense primer No. 8:
5'-pTTAAGTTAAGAGAAAGTGCT-3'
(Sequence ID No. 9)
A 5 µl solution of the human placental cDNA library λgt11 (CLONTECH Laboratories, Inc.) and 45 µl of distilled water were mixed. After incubation at 95°C for 5 minutes, the mixture was immediately ice cooled. Two primers (Nos. 5 and 6 above, 50 pmol of each) were added, and PCR carried out using 1 unit of Vent_{R} DNA Polymerase in the presence of 20 mM MgSO₄, in which a series of reactions at 94°C for 1 minute, 55°C for 1 minute and 72°C for 1 minute 10 seconds was repeated in 50 cycles. To the reaction mixture were added two other primers (Nos. 6 and 7 above, 50 pmol of each), and PCR was carried out in the same manner as above. The PCR product was separated by electrophoresis on 1.2% agarose gel; an amplified DNA fragment was seen at a position corresponding to the size (1076 bp) expected from the nucleotide sequence of cathepsin S of human placental origin. This DNA fragment was recovered from the gel and subcloned to the plasmid vector pT7Blue(R)T-Vector (produced by Novagen Company). The nucleotide sequence of the cDNA portion was determined by the dideoxynucleotide synthetic chain termination method [J. Messing et al., Nucleic Acids Res., 9, 309 (1981)]. The plasmid containing this cDNA fragment was named pHCS-2.

### Example 1: Preparation (1) of recombinant DNA for expression of human cathepsin L gene in animal cells

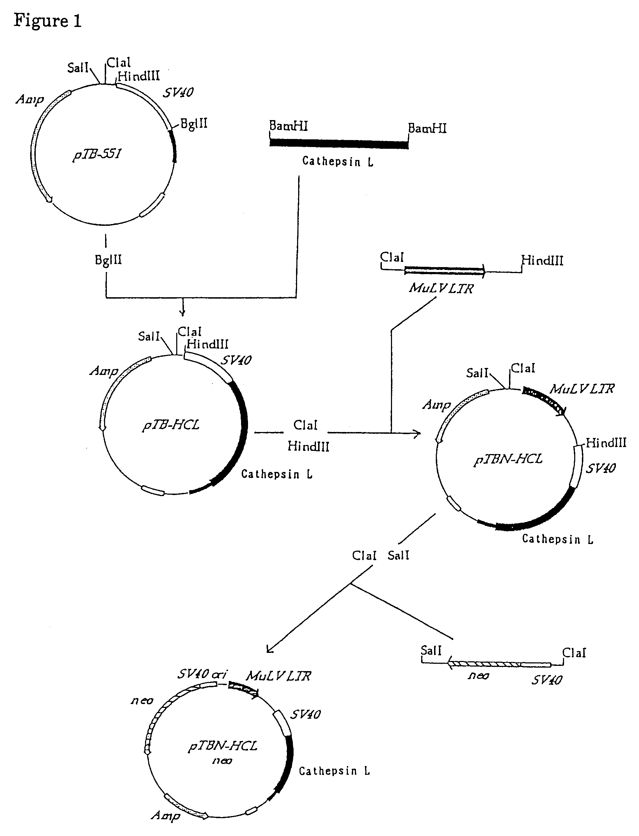

After the plasmid pHCL-5, prepared in Reference Example 1, was digested with restriction enzyme BamHI, a fragment of human cathepsin L cDNA was recovered by agarose gel electrophoresis. Next, this cDNA fragment was inserted into the restriction enzyme BglII site of the vector pTB551 for transient expression in animal cells [prepared by converting the EcoRI site to BglII site in the plasmid pTB389 described by Ono et al. in Science, 236, 1116 (1989)] by the action of T4 DNA ligase and ATP, to yield the expression plasmid pTB-HCL. MuLV-LTR was inserted between the restriction enzyme HindIII and ClaI sites of pTB-HCL to yield the expression plasmid pTBN-HCL (Figure 1).

### Example 2: Expression (1) of human cathepsin L gene in animal cells

To a 6-cm petri dish was added 4 ml of a complete medium [DMEM medium containing 5% (v/v) FCS (fetal calf serum)], and 3 × 10⁵ COS-7 cells were inoculated. This culture broth was incubated at 37°C overnight in the presence of 5% carbon dioxide, followed by medium replacement with fresh medium.

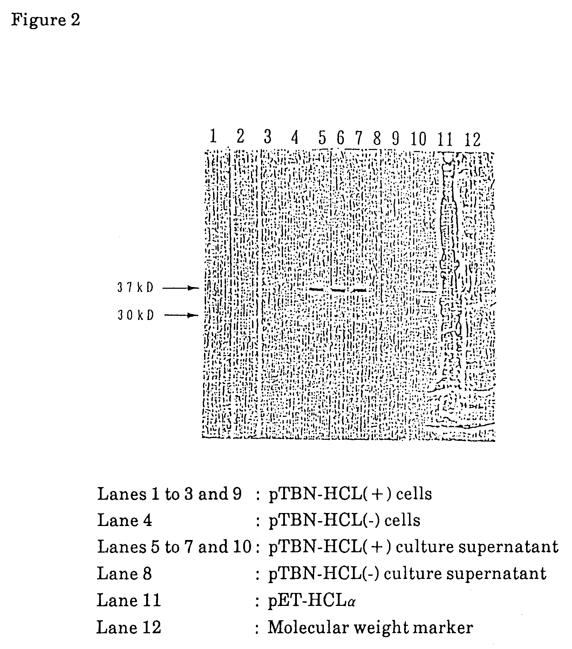

To 20 µg of the plasmid described in Example 1 (pTBN-HCL), 50 µl of a 2.5 M calcium chloride solution (sterilized) was added, and sterile water was added for a final volume of 500 µl. This solution was added to 500 µl of a 2 × HBS solution (280 mM sodium chloride, 1.5 mM disodium monohydrogen phosphate, 50 mM HEPES, pH 7.05) to form a DNA-calcium phosphate precipitate. Two hours after the medium replacement, 500 µl of this precipitate suspension was added to cells drop by drop, and this mixture was cultured at 37°C in the presence of 5% carbon dioxide. Four hours later the medium was removed, and 1.5 ml of a 15% glycerol/1 × HBS solution was added. After the mixture was kept standing for 30 seconds, the glycerol/1 × HBS solution was removed, and the cells adhering to the petri dish were washed with DMEM medium (serum-free). After 4 ml of the complete medium was added, cultivation was continued under the above conditions, and 24 hours later, the medium was replaced with serum-free ASF104 medium (produced by Ajinomoto Co., Inc.), followed by two more days of cultivation. The culture supernatant was subjected to western blot analysis using the antiserum to human cathepsin L prepared in Reference Example 3; in response to the antiserum, unique bands appeared, which had molecular weights of about 40,000 to 30,000 and lower molecular weights. These were identified as human pro-cathepsin L and decomposed products thereof, estimated from these molecular weights (Figure 2).

### Example 3: Determination of expressed human cathepsin L activity

The culture supernatant of COS-7 transformant prepared in Example 2 was concentrated about 20-fold with Centricut (produced by Kurabo Industries, Ltd., cut-off molecular weight = 10000) and assayed for cathepsin L enzyme activity, according to the method of A. J. Barrett and H. Kirschke [Methods in Enzymology, 80, 535 (1981)]. To 25 µl of this concentrated culture supernatant were added 25 µl of 1 M sodium formate buffer (pH 3.0) and 10 µl of 0.1 M DTT, and water was added for a final volume of 250 µl, followed by incubation at 37°C for 30 seconds. After 125 µl of activating buffer (340 mM sodium acetate, 60 mM acetic acid, 4 mM EDTA disodium, pH 5.5) was added, the mixture was kept standing at 30°C for 1 minute for activation. The enzyme reaction was carried out at 30°C for 10 minutes in the presence of 125 µl of a synthetic substrate solution [20 µM Z-Phe-Arg-NMec (Z: benzyloxycarbonyl-, NMec: -7-(4-methyl)coumarylamide)], and stopped by the addition of 500 µl of a reaction stopper (100 mM sodium monochloroacetate, 30 mM sodium acetate, 70 mM acetic acid, pH 4.3). For a control experiment, a culture supernatant of COS-7 cells not expressing cathepsin L was used as an enzyme solution. After termination of the reaction, 100 µl of the reaction mixture was injected into a 96-well fluoroplate (produced by Labo Systems), and the fluorescence intensity of free 7-amino-4-methylcoumarin was determined at a wavelength of 450 nm (excitation wavelength = 365 nm) using a fluorometer FCA (produced by Baxter). In assessing the effects of the following enzyme inhibitors on the present enzyme activity, the inhibitor was added after the above enzyme activating operation. The enzyme inhibitors used were 10 µM leupeptin (cysteine protease inhibitor), 10 µM (p-amidinophenyl)methanesulfonyl fluoride hydrochloride (APMSF: serine protease inhibitor), 1 µM Z-Phe-Phe-CHN₂ (cathepsin L inhibitor, -CHN₂: -diazomethane) and 1 µM pepstatin A (aspartic acid protease inhibitor).

The assay detected cathepsin L activity in the culture supernatant of the COS-7 cells transformed with human cathepsin L gene. The enzyme activities obtained in the presence of enzyme inhibitors leupeptin, APMSF, Z-Phe-Phe-CHN₂ and pepstatin A were 3.4%, 79%, 6.3% and 99%, respectively, in comparison with the inhibitor-free control. These findings provide strong evidence that the identity of the product expressed in the COS-7 cells is human cathepsin L.

### Example 4: Preparation (2) of recombinant DNA for expression of human cathepsin L gene in animal cells

To obtain an animal cell line that stably expresses human cathepsin L, the drug resistance marker neo gene was inserted into the vector pTBN-HCL described in Example 1. Namely, a fragment comprising the SV40 early promoter and the neo gene was inserted between the restriction enzyme ClaI and SalI sites of the plasmid pTBN-HCL to yield the plasmid pTBN-HCLneo (Figure 1).

### Example 5: Expression (2) of human cathepsin L gene in animal cells

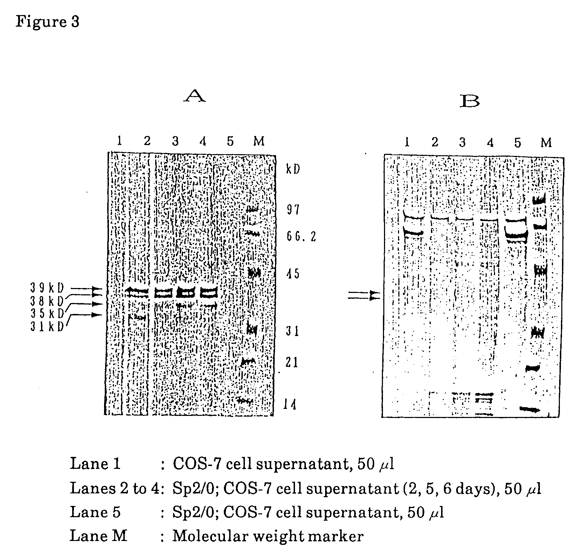

Using the plasmid prepared in Example 4 (pTBN-HCLneo), mouse myeloma Sp2/0 cells were transformed as follows: Sp2/0 cells, cultivated in an ASF104 medium supplemented with 5% FCS (5% FCS/ASF), were suspended in PBS(-) [the same as Dullbecco's PBS but CaCl₂ and MgCl₂ are removed], so that the concentration of the medium became 1 × 10⁷ cells/ml. 500 µl of this cell suspension was injected into a cuvette, 10 µg of said plasmid DNA was added, and the mixture was kept standing on ice for 5 minutes. This liquid was pulsated at 125 µF and 300 V using a gene pulsar (produced by Bio-Rad Laboratories) and then kept standing on ice again for 10 minutes. The liquid was then transferred to 10 ml of 5% FCS/ASF104 medium and cultured at 37°C in the presence of 5% carbon dioxide. 48 hours later the culture was transferred to selection medium (5% FCS/ASF104 medium containing 200 µg/ml G418) and cultured on a 24-well plate for 2 weeks. A number of colonies were detected, each of which was transferred to an ASF104 medium containing 200 µg/ml G418 and cultured, followed by western blot analysis of the culture supernatant using the antiserum to human cathepsin L prepared in Reference Example 3. In response to the antiserum, unique bands appeared having molecular weights of about 40,000 to 30,000 and lower molecular weights; estimating from these molecular weights, they were identified as a human pro-cathepsin L and processing product thereof (Figure 3). The culture supernatant was assayed for cathepsin L enzyme activity, according to the method described in Example 3; human cathepsin L activity was detected. These findings confirm that transformant mouse myeloma cells expressing cathepsin L were obtained, which were designated as mouse myeloma Sp-HCL26.

### Example 6: Purification of human cathepsin L

The strain obtained in Example 5, showing high expression of cathepsin L, was cultured in 20 ml of ASF104 medium supplemented with 10% FCS and 200 µg/ml G418, after which it was transferred to 50 ml of serum-free selection medium (ASF104 medium supplemented with 200 µg/ml G418) and cultured for 5 days. After the culture supernatant was applied to a column of CM-Sephadex C-50 (25 × 4.4 cm), the column was washed with buffer A (20 mM sodium acetate, 1 mM EDTA, pH 5.5), followed by elution on a sodium chloride (NaCl) density gradient from 0 to 1 M, to elute human cathepsin L near an NaCl concentration of about 0.4 M. This fraction was applied to a Mono S column (HR5/5) of an FPLC system (Pharmacia), followed by column washing and human cathepsin L elution in the same manner as above. The human cathepsin L fraction, eluted near an NaCl concentration of about 0.36 M, was concentrated to yield a purified preparation.

### Example 7: Action of human cathepsin L on intervertebral pulp

Aqueous solution compositions containing human cathepsin L at 200, 400, 800 or 1600milliunits were prepared.

0.01 ml of the composition was injected in the lumbar intervertebral pulp of rabbits (weighing 2.5 to 3 kg) via a posterior lateral approach under radiographic observation. One week later radiography revealed stenosis in lumbar intervertebral disks in the rabbits receiving cathepsin L at doses of 800milliunits or more.

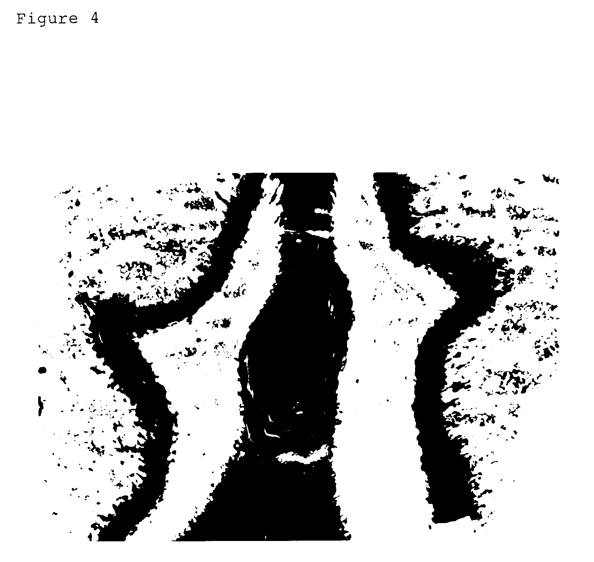

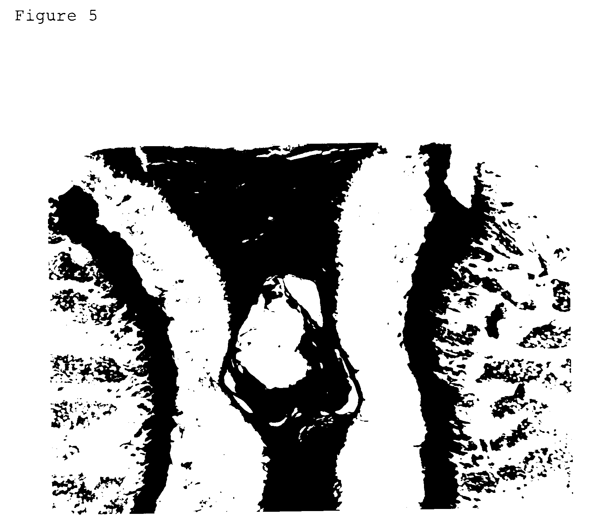

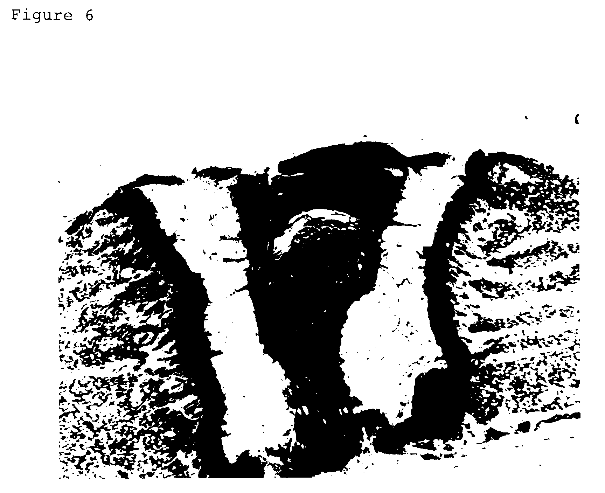

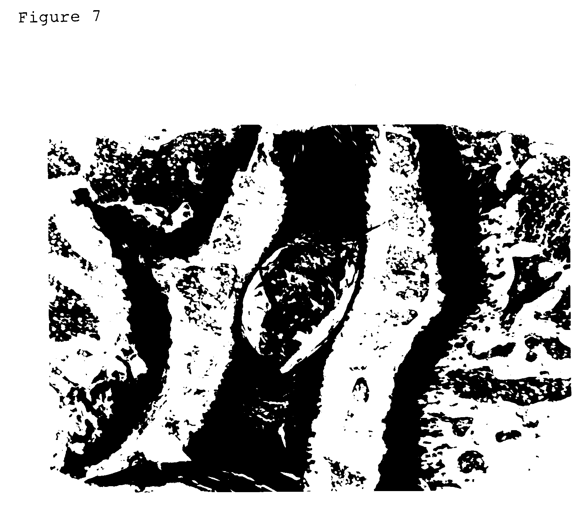

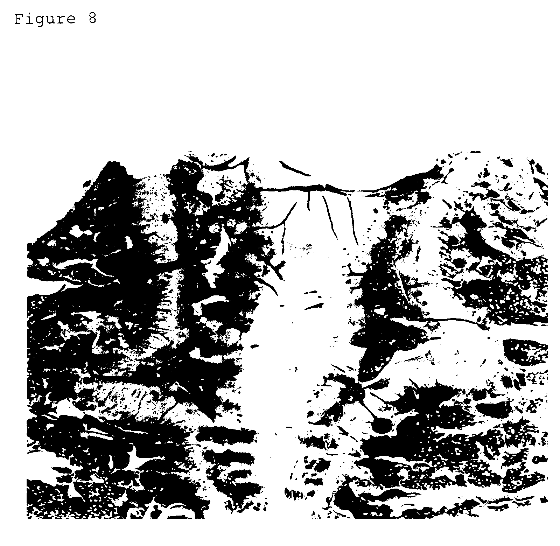

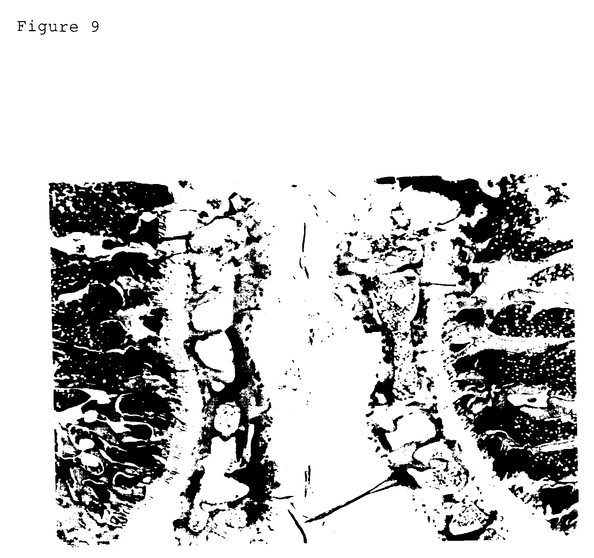

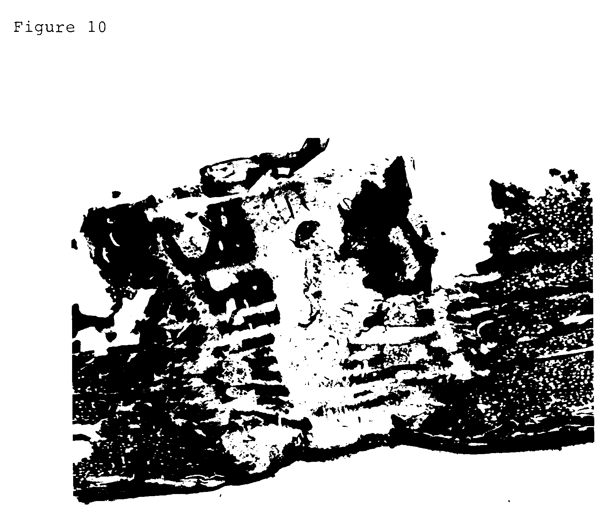

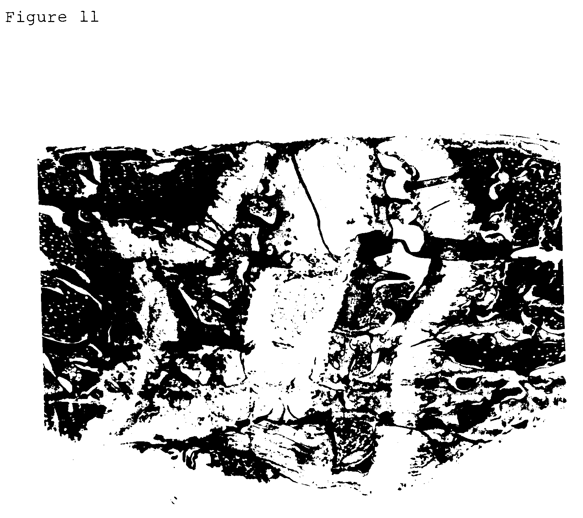

For histologic examination, the rabbits were sacrificed by exsanguination under anesthesia, and the lumbar vertebrae were collected from the site of cathepsin L injection. The bone tissue was fixed in formalin and decalcified with EDTA, after which it was trimmed to prepare for longitudinal sections and embedded in paraffin. Next, 5-micron thin sections were prepared using a microtome and stained with toluidine blue (which selectively stains cartilage, Figures 4 through 7) and hematoxylin-eosin (Figures 8 through 11), after which these sectional specimens were observed using a light microscope. The pulp was slightly lysed at a dose of 800milliunits (Figures 5 and 9) and almost totally lysed at a dose of 1600milliunits (Figures 4 and 8). However, at a dose of 400milliunits (Figures 6 and 10) or 200milliunits (Figures 7 and 11), no such reduction in pulp was observed, as in the control group receiving a physiological saline injection.

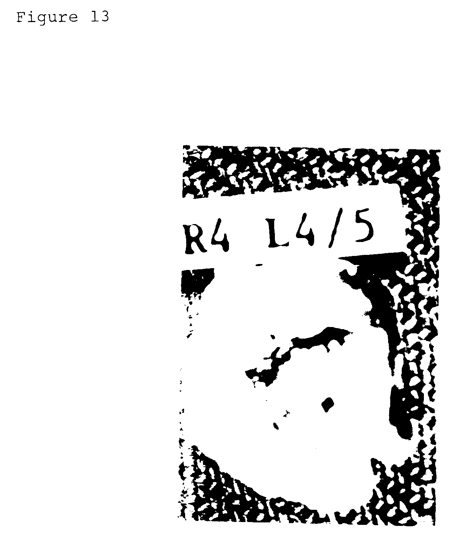

One week after the 800milliunits cathepsin L injection, pulp lysis occurred in the rabbit intervertebral disks, affecting the fibrous ring (Figure 13).

### Example 8: Purification (2) of human cathepsin L

The single clone Sp-HCL26 obtained in Example 5, showing high expression of human cathepsin L, was cultured in 1.8 liter of selection medium (ASF104 medium supplemented with 3% FCS and 600 µg/ml G418), after which it was centrifuged. The resulting culture supernatant was recovered and concentrated to 370 ml using a Culture Flow MRL unit (produced by Asahi Medical Co., Ltd.). To the concentrate was added ammonium sulfate to 80% saturation, followed by stirring at 4°C for 2 hours and subsequent centrifugation at 17,000 × g for 15 minutes, to yield a precipitate, which was suspended in 25 ml of buffer A (20 mM sodium acetate, 1 mM EDTA, pH 5.5) and dialysed against buffer A overnight.

The dialysate was then applied to a column of CM-Sepharose Fast Flow (3.0 × 30 cm, Pharmacia LKB Biotechnology). After column washing with buffer A in an amount five times the bed volume, human cathepsin L was eluted with buffer A containing 0.4 M NaCl. The eluate was concentrated using a Centricut U-10 (produced by Kurabo Industries, Ltd.) and applied to a column of Sephacryl S-200 HR (2.5 × 100 cm, Pharmacia LKB Biotechnology), equilibrated with buffer A containing 0.9% NaCl, followed by gel filtration. The fraction containing human cathepsin L was recovered and dialysed against buffer A overnight. The dialysate was applied to a column of CM-Sephadex C-50 (1.6 × 20 cm, Pharmacia LKB Biotechnology). After column washing with buffer A in an amount three times the bed volume, human cathepsin L was eluted on a linear sodium chloride density gradient (final concentration 300 mM). The human cathepsin L fraction eluted at a concentration of about 160 mM was concentrated using a Centricut U-10 and then subjected to the same procedure of gel filtration chromatography as above. The human cathepsin L fraction was recovered, and 50% saturated with ammonium sulfate, after which it was applied to a HiLoad Phenyl-Sepharose High Performance column (16/10, Pharmacia LKB Biotechnology), equilibrated with buffer A containing 50% saturated ammonium sulfate. After column washing with buffer A containing 50% saturated ammonium sulfate, the column was eluted with buffer A containing 50% (v/v) ethylene glycol to yield 0.67 mg of a purified human cathepsin L. A 17 µg sample of this preparation was subjected to polyacrylamide gel electrophoresis. A band of a 33 kD mature region of cathepsin L alone appeared. Although the human cathepsin L was secreted in the form of an about 41 kD precursor in the culture supernatant, it was processed into the about 33 kD highly active mature protein as a result of removal of the FCS-derived albumin in the medium during the purification process.

### Example 9: Preparation of recombinant DNA for expression of human cathepsin S gene in animal cells

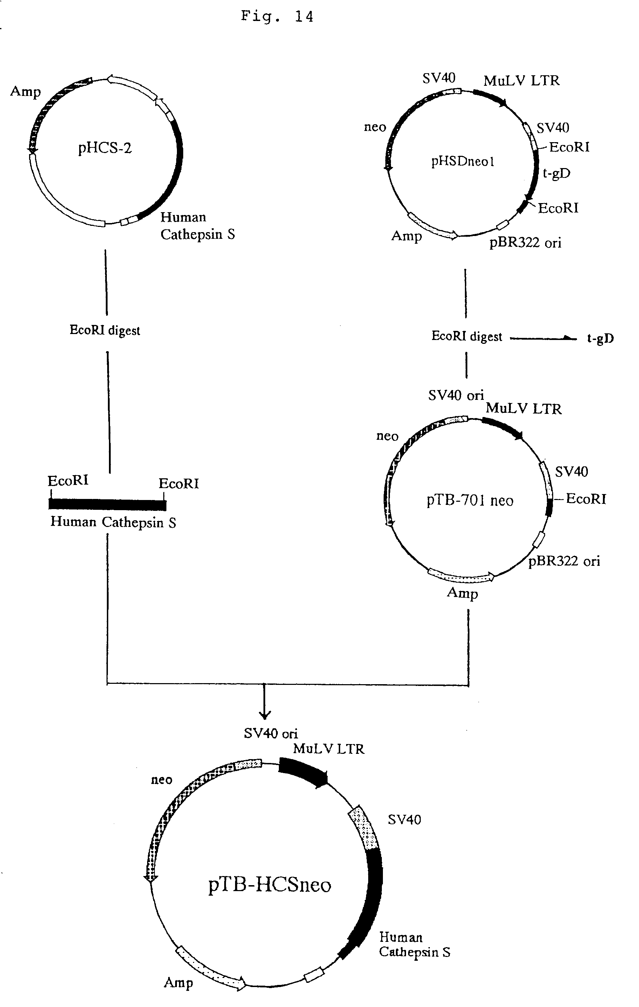

After the plasmid pHCS-2, described in Reference Example 4, was digested with the restriction enzyme EcoRI, a fragment of human cathepsin S cDNA was recovered by agarose gel electrophoresis. Next, this cDNA fragment was inserted into the restriction enzyme BglII site of the vector pTB701neo for transient expression in animal cells (resulting from removal of the gD gene from the plasmid pHSDneo1 described in Example 5 (Japanese Patent Unexamined Publication No. 117399/1992)) by the action of T4 DNA ligase and ATP, to yield the expression plasmid pTBN-HCSneo (Figure 14).

### Example 10: Expression of human cathepsin S gene in animal cells

Using the plasmid described in Example 9 (pTBN-HCSneo), mouse myeloma Sp2/0 cells were transformed as follows: Sp2/0 cells, cultivated in an ASF104 medium supplemented with 5% FCS (5% FCS/ASF), were suspended in PBS(-) [the same as Dullbecco's PBS but CaCl₂ and MgCl₂ were removed] to 1 × 10⁷ cells/ml. 500 µl of this cell suspension was injected to a cuvette, 10 µg of said plasmid DNA was added, and the mixture kept standing on ice for 5 minutes. This liquid was pulsated at 125 µF and 300 V using a gene pulsar (produced by Bio-Rad Laboratories) and kept standing on ice for a further 10 minutes. This liquid was transferred to 10 ml of 5% FCS/ASF104 medium and cultured at 37°C in the presence of 5% carbon dioxide. 48 hours later the culture was transferred to selection medium (5% FCS/ASF104 medium containing 200 µg/ml G418) and cultured on a 24-well plate for 2 weeks. A number of colonies were detected, each of which was transferred to an ASF104 medium containing 200 µg/ml G418 and subcultured, followed by western blot analysis of the culture supernatant using the antiserum to human cathepsin L (antibody showing cross reaction with human cathepsin S) prepared in Reference Example 3. In response to the antiserum, unique bands appeared having molecular weights of about 38,000 to about 23,000 and lower; they were identified as a human pro-cathepsin S and processing products thereof, as estimated from the molecular weights. The culture supernatant was assayed for cathepsin S activity, in accordance with the method described in Example 3; human cathepsin S activity was detected.

### Example 11: Purification of human cathepsin S

The single clone Sp-HCS30 obtained in Example 10, showing high expression of human cathepsin S, was cultured in 2.0 liter of a selection medium (ASP104 medium supplemented with 3% FCS and 600 µg/ml G418), after which it was centrifuged, and the culture supernatant recovered. Using this culture supernatant as a starting material, human cathepsin S was purified, in accordance with the method described in Example 8, to yield about 0.5 mg of a purified preparation.

### Example 12: Action of human cathepsin S on intervertebral pulp

Human cathepsin S was injected in the rabbit lumbar intervertebral pulp as directed in Example 7. As in the case of human cathepsin L, intervertebral disk anastomosis was noted.

SEQUENCE LISTING
GENERAL INFORMATION
APPLICANT:
NAME: Takeda Chemical Industries, Ltd.
STREET: 1-1, Doshomachi 4-chome, Chuo-ku,
CITY: Osaka
COUNTRY: Japan
POSTAL CODE: 541
TELEPHONE: (06) 300-6778
TELEFAX: (81) -6-300-6601
TELEX: 63404
TITLE OF INVENTION: Production of Human Cathepsin and Its Use
NUMBER OF SEQUENCES: 9
COMPUTER READABLE FORM:
MEDIUM TYPE: diskette-3.5 inch 1.0 mb
COMPUTER: IBM PC compatible
OPERATING SYSTEM: PC-DOS/MS-DOS
SOFTWARE: IBM Dos Version 4.01

Sequence Listing
SEQ ID NO : 1
Sequence length : 20
Sequence type : Nucleic acid
Strandedness : Single
Topology : Linear
Molecule type : Other nucleic acid (chemically synthesized DNA)
Antisense : No
Sequence:
TTTTCAGGGG GCAGTAAGAT 20

SEQ ID NO: 2
Sequence length : 28
Sequence type : Nucleic acid
Strandedness : Single
Topology : Linear
Molecule type : Other nucleic acid (chemically synthesized DNA)
Antisense : No
Sequence:
CCGGATCCGG CTTTTTAGGA TTGGTCTA 28

SEQ ID NO : 3
Sequence length : 20
Sequence type : Nucleic acid
Strandedness : Single
Topology : Linear
Molecule type : Other nucleic acid (chemically synthesized DNA)
Antisense : Yes
Sequence:
GGGGGCTGG TAGACTGAAGA 20

SEQ ID NO : 4
Sequence length : 28
Sequence type : Nucleic acid
Strandedness : Single
Topology : Linear

Molecule type : Other nucleic acid (chemically synthesized DNA)
Antisense : Yes
Sequence:
CCGGATCCAT TCCTCCCATG CATGCGCC 28

SEQ ID NO : 5
Sequence length : 12
Sequence type : Nucleic acid
Strandedness : Single
Topology : Linear
Molecule type : Other nucleic acid (chemically synthesized DNA)
Antisense : No
Sequence:
CCCGGATCCG GG 12

SEQ ID NO : 6
Sequence length : 20
Sequence type : Nucleic acid
Strandedness : Single
Topology : Linear
Molecule type : Other nucleic acid (chemically synthesized DNA)
Antisense : No
Sequence:
TAATTCAAGG ACTCTTACTG 20

SEQ ID NO : 7
Sequence length : 28
Sequence type : Nucleic acid
Strandedness : Single
Topology : Linear
Molecule type : Other nucleic acid (chemically synthesized DNA)
Antisense : No
Sequence:
GGAATTCCTA GGAGCAACTG CTGGTTCT 28

SEQ ID NO : 8
Sequence length : 28
Sequence type : Nucleic acid
Strandedness : Single
Topology : Linear
Molecule type : Other nucleic acid (chemically synthesized DNA)
Antisense : Yes
Sequence:
GGAATTCCTC ATATTTCATT GATTTGTT 28

SEQ ID NO : 9
Sequence length : 20
Sequence type : Nucleic acid
Strandedness : Single
Topology : Linear
Molecule type : Other nucleic acid (chemically synthesized DNA)
Antisense : Yes
Sequence:
TTAAGTTAAG AGAAAGTGCT 20

## Claims

1. A method of producing human cathepsin, wherein animal cells carrying a DNA encoding human cathepsin are cultured to produce and accumulate said enzyme in the culture broth, which is then harvested.

2. A mouse myeloma cell carrying a DNA encoding human cathepsin.

3. The mouse myeloma cell as claimed in claim 2, wherein cathepsin is cathepsin L.

4. The mouse myeloma cell as claimed in claim 2, wherein cathepsin is cathepsin S.

5. The mouse myeloma cell as claimed in claim 3, wherein the cell is mouse myeloma cell Sp-HCL26 (FERM BP-3902) or Sp-HCL26-1 (FERM BP-4288).

6. The mouse myeloma cell as claimed in claim 4, wherein the cell is mouse myeloma cell Sp-HCS30 (FERM BP-4289).

7. Use of human cathepsin for the manufacture of a medicament to be used for treating or improving mammalian intervertebral disk hernia.

8. The use as claimed in claim 7, wherein said human cathepsin is human cathepsin L.

9. The use as claimed in claim 7, wherein said human cathepsin is human cathepsin S.

10. The use as claimed in claim 7, wherein said medicament is for direct injection to the intervertebral pulp.
